# EUROPEAN PATENT SPECIFICATION

(11) **EP 3 485 262 B1**
(45) Date of publication and mention of the grant of the patent: **11.11.2020**
(21) Application number: 17749752.6
(22) Date of filing: 10.07.2017
(51) Int. Cl.: G01N 27/12, G01N 33/00

(54) **SENSOR FOR DETECTING AMMONIA GAS OR AMMONIA VAPOUR, METHOD OF MANUFACTURING OF SAID SENSOR AND ITS USE**
SENSOR ZUM NACHWEIS VON AMMONIAKDAMPF ODER -GAS, VERFAHREN ZU DESSEN HERSTELLUNG UND DESSEN VERWENDUNG
CAPTEUR POUR LA MESURE DE VAPEURS OU GAS D'AMMONIAC, METHODE DE SA PRODUCTION ET SON UTILISATION

(30) Priority: 12.07.2016 IT 201600072363
(43) Date of publication of application: 22.05.2019
(73) Proprietor: Fondazione Istituto Italiano di Tecnologia, 16163 Genova (IT)
(72) Inventor: BAYER, Ilker, 16162 Genova (IT); ATHANASIOU, Athanasia, 16014 Ceranesi (GE) (IT); PAPADOPOULOU, Paraskevi, 16122 Genova (IT); MORSELLI, Davide, 16125 Genova (IT)
(74) Representative: Vitillo, Giuseppe
(86) International application number: PCT/IB2017/054137
(87) International publication number: WO 2018/011694

(56) References cited:
- EP-A2- 0 398 286
- WO-A1-2016/102028
- US-A- 4 142 400
- V Strijkova ET AL: "Vacuum Deposited Polyimide Layers as Humidity Sensors *", Bulg. J. Phys, 30 September 2013 (2013-09-30), pages 224-228, XP055376367, Retrieved from the Internet: URL:http://www.bjp-bg.com/papers/bjp2013_3 _224-228.pdf [retrieved on 2017-05-29]
- MEHMET DOKMECI ET AL: "A HIGH-SENSITIVITY POLYIMIDE HUMIDITY SENSOR FOR MONITORING HERME TIC MI CR 0 PA CKA GE S", MICRO ELECTRO MECHANICAL SYSTEMS, 1999. MEMS '99. TWELFTH IEEE INTERNATIONAL CONFERENCE ON, 21 January 1999 (1999-01-21), pages 279-284, XP055376691,
- HIRATA M ET AL: "Characteristics of an organic semiconductor polyaniline film as a sensor for NH"3 gas", SENSORS AND ACTUATORS A: PHYSICAL, ELSEVIER BV, NL, vol. 40, no. 2, 1 February 1994 (1994-02-01), pages 159-163, XP026529944, ISSN: 0924-4247, DOI: 10.1016/0924-4247(94)85024-0 [retrieved on 1994-02-01]
- GIUSEPPE RAGOSTA ET AL: "Effect of the chemical structure of aromatic polyimides on their thermal aging, relaxation behavior and mechanical properties", JOURNAL OF MATERIALS SCIENCE, KLUWER ACADEMIC PUBLISHERS, BO, vol. 47, no. 6, 11 November 2011 (2011-11-11), pages 2637-2647, XP035004022, ISSN: 1573-4803, DOI: 10.1007/S10853-011-6089-0
- VIEHBECK A ET AL: "ELECTROCHEMICAL PROPERTIES OF POLYIMIDES AND RELATED IMIDE COMPOUNDS", JOURNAL OF THE ELECTROCHEMICAL SOCIETY, ELECTROCHEMICAL SOCIETY, INC, US, vol. 137, no. 5, 1 May 1990 (1990-05-01), pages 1460-1466, XP000235359, ISSN: 0013-4651
- WENGUO NING ET AL: "The effects of cure temperature history on the stability of polyimide films", JOURNAL OF SEMICONDUCTORS, vol. 34, no. 6, 1 June 2013 (2013-06-01), page 063003, XP055411248, GB; CN ISSN: 1674-4926, DOI: 10.1088/1674-4926/34/6/063003

## Description

### Technical field

The present invention relates to a sensor for detecting the presence of ammonia gas and/or ammonia vapour, a method for manufacturing said sensor, and the use of said sensor.

### Background art

Several types of sensors are known in the art for detecting the presence of ammonia gas and/or ammonia vapours.

The various technical solutions known in the art for detecting the presence of ammonia gas or vapours include the use of electrically conductive polymers, e.g. polypyrrole, polyaniline, co-polyesters characterized by systems of conjugated double bonds, histidine-substituted perylene diimide (abbreviated as PDI-HIS).

Furthermore, nanostructured metal oxides (MoO₃, W₁₈O₄₉, In2O3, ZnO, α-Fe₂O₃) have also been used for detecting ammonia gas.

In particular, nanocomposites based on conductive polymers and metal oxides represent one of most important families of materials typically in use for ammonia detection. Finally, carbon-based nanostructures can also be used for detecting the presence of ammonia; more specifically, carbon and graphene nanotubes (and possibly also nanocomposites based on such materials).

US 4 142 400 A discloses an element for sensing the presence of nitrogen dioxide gas in the environment. The element comprises a film of a pyrolyzed polyaromatic polymer having semi-conductive properties which is mounted on an electrically non-conductive base. Electrical leads are attached at spaced locations to the film and are connected in an electrical circuit with a signalling or control mechanism. The presence of nitrogen dioxide gas will cause a change in the electrical characteristics of the film to thereby activate the mechanism. A heating element can be associated with the base to insure constant temperature and humidity conditions.

### Summary of the invention

It is one object of the present invention to provide a sensor for detecting ammonia gas and/or ammonia vapour, which ensures a reliable detection of ammonia from aqueous solutions, even at low-molarity ammonia concentrations.

It is another object of the invention to provide a method for manufacturing such a sensor.

It is a further object of the invention to provide a use of such a sensor for detecting ammonia gas and/or ammonia vapour.

According to the present invention, this and other objects are achieved through a sensor having the technical features set out in the appended independent claims.

It is to be understood that the appended claims are an integral part of the technical teachings provided in the following detailed description of the invention. In particular, the appended dependent claims define some preferred exemplary embodiments of the present invention, which include some optional technical features.

In some illustrative embodiments, the sensor according to the present invention has numerous advantageous aspects, including:
- the sensor can operate at room temperature;
- simple manufacturing process, not requiring the use of any additive or coating;
- it includes light materials;
- the possibility of conducting a low-temperature heat treatment, using low-cost materials and obtaining a fast preparation leads to low energy consumption;
- no need for using nanoparticles or carbon structures for ammonia detection;
- the sensor can also detect low-molarity (e.g. as low as 3.5 mM) ammonia gas or vapour;
- the sensitive material employed in the sensor can be easily deposited on different types of electrically insulating substrates, whether rigid or flexible, e.g. glass, quartz, textile, paper, etc.;
- the sensor can be easily implemented also with porous substrates; and
- the sensitive material employed in the sensor offers biocompatibility and excellent thermal and mechanical properties, as well as very good chemical resistance, even at high operating temperatures, e.g. up to 400°C.

Further features and advantages of the present invention will become apparent from the following detailed description, which is supplied by way of non-limiting example with particular reference to the annexed drawings, which will be summarized below.

### Brief description of the drawings

Figure 1 is a schematic view of a sensor manufactured in accordance with one exemplary embodiment of the present invention, which is shown herein incorporated into a system;
Figure 2 is a Cartesian-axis graph that shows the trend of the current intensity as a function of time, as detected in a sensor manufactured according to one exemplary embodiment of the present invention. Said current intensity is detected with the sensor interacting with different concentrations (molarities) of ammonia gas and/or vapours.
Figure 3 is a diagram of a control circuit that can be used in a system comprising a sensor manufactured in accordance with one exemplary embodiment of the present invention.

### Detailed description of the invention

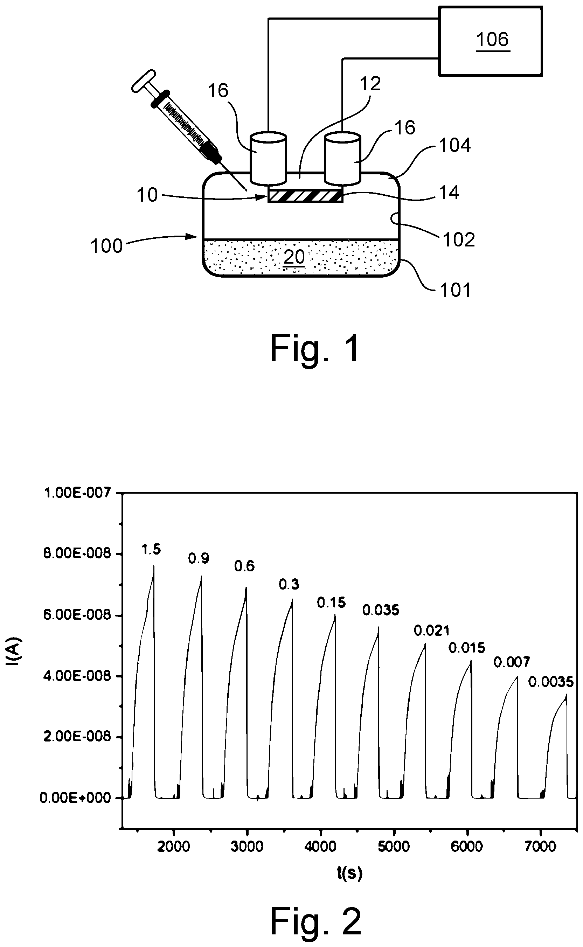

With reference to Figure 1, there is shown a sensor manufactured in accordance with one exemplary embodiment of the present invention.

The sensor, designated as a whole as **10**, is configured for detecting the presence of ammonia gas and/or ammonia vapour.

The sensor **10** comprises a substrate **12** whereon a polymeric layer **14** is deposited, which has electrically conductive properties when that region adsorbs a quantity of ammonia. In the illustrated embodiment, the polymeric layer **14** entirely coats one face of the substrate **12**. In further alternative embodiments, however, the polymeric layer **14** may coat the substrate **12** just partially.

The sensor **10** also comprises a pair of electrodes **16** situated in contact with the polymeric layer **14** and configured for detecting a variation in the electric resistance of said polymeric layer **14**. This electric resistance variation is caused by the presence of said ammonia gas or vapour.

As aforementioned, the polymeric layer **14** comprises (or consists of) a polyimide, i.e. a polymer made up of monomers containing imide groups.

Experimental tests have surprisingly shown that, when the polyimide adsorbs a quantity of ammonia gas or vapour (e.g. in an environment where ammonia is present in an aqueous solution, designated as **20** in Figure 1), it exhibits electrically conductive properties. In particular, it was found out that, following adsorption of ammonia gas or vapour, the electric resistance of the polyimide changes according to the adsorbed quantity of said gas or vapour. More in detail, said electric resistance decreases as the concentration of the ammonia gas or vapour interacting with the polyimide increases.

The variation in the electric resistance of the polyimide is a function of the concentration of the ammonia gas and/or vapour developed by the ammonia solution, even at low molarity. When the sensor **10** is removed from the ammonia-containing environment and returns into ambient conditions, the electric resistance of the polyimide that constitutes the polymeric layer **14** also goes back to higher values. The sensor **10** described herein is therefore configured for detecting ammonia gas or vapour starting from solutions having different molarity.

More in detail, it was surprisingly verified that, when the polyimide is exposed to the vapours developed by the ammonia solution, diffusion processes occur which result in a chemical modification of the structure of the polyimide due to interaction with ammonia. Some functional groups of the polyimide react with ammonia as shown in the following diagram.

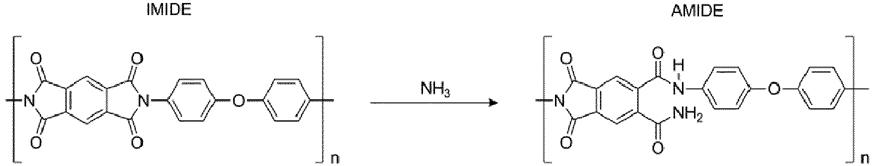

The use of a polymeric layer **14** comprising or, more preferably, consisting of polyimide as a sensitive element of the sensor **10** turns out to be particularly innovative and inventive due to the fact that, according to the prior art, polyimide has been used so far only as a substrate for ammonia gas and/or vapour sensors. The present invention, instead, provides a sensor **10** that utilizes polyimide for the ammonia-sensing polymeric layer **14**, as opposed to proposing the use thereof only as a support substrate for the sensitive element of the sensor.

Preferably, the sensing polymeric layer **14** consists of polyimide obtained by polycondensation, i.e. the imide functional group is formed during the polymerization reaction.

In the embodiment illustrated herein by way of example, the polyimide that forms at least part of the polymeric layer **14** is produced, in a *per se* known manner, starting from pyromellitic dianhydride-co-4,4'-oxydianiline and aminic acid (said polyimide being commonly referred to by the acronym PMDA-ODA).

By way of non-limiting example, preparation of the sensing polymeric layer **14** occurs through the following steps:
- providing a solution including a value in the range of 10% to 20% (e.g. 15%) in weight of pyromellitic dianhydride-co-4,4'-oxydianiline and aminic acid in N-methyl-2-pyrrolidone (commonly known as NMP);
- diluting said solution into a mixture of dimethylacetamide (commonly known as DMA) and an organic polar solvent, e.g. acetone, (at a concentration between 1:0.5 and 1:2, preferably 1:1), so as to obtain a solution with 5% to 20%, preferably 10%, of PMDA-ODA;
- depositing said solution on the substrate **12**, e.g. glass;
- leaving said solution to dry on the substrate **12** for one or more hours; and
- polymerizing said solution dried on the substrate **12** by oven heating.

Polymerization generally occurs by heating the solution deposited and dried on the substrate **12** in an oven, particularly in two consecutive steps. A first step is shorter, e.g. approx. 15-60 minutes, and occurs at a lower temperature, e.g. 60°C to 100°C. A second step lasts longer, e.g. approx. 120-200 minutes, and occurs at a higher temperature, e.g. 170°C to 250°C. Preferably, the first step lasts about 20 minutes at approx. 80°C, and the second step lasts about 150 minutes at approx. 220°C. In this manner, full polymerization of the PMDA-ODA is achieved.

The solution can be deposited on different types of electrically insulating substrates **12**, whether rigid or flexible.

According to one advantageous embodiment, the material of the substrate **12** is, for example, glass or quartz.

According to another advantageous embodiment, the substrate **12** may be made of fibrous material, e.g. textile or paper.

The solution can be deposited on the substrate **12** in different ways, e.g. by dip-coating, or by deposition of liquid drops (drop-casting), or by means of other techniques known as spray-coating and spin-coating.

The Applicant carried out numerous experimental tests on the sensor, which proved to be particularly effective compared with those made in accordance with the prior art.

In particular, as is visible in the embodiment shown in Figure 1, a system **100** comprises the sensor **10**. The system **100** further comprises a container **101** that defines an internal chamber **102**, in which the sensor **10** is mounted. In particular, the sensor **10** is mounted near the top of the chamber **102**. For example, the sensor **10** may be constrained to an element for closing the container **101**, which encloses the chamber **102**. Advantageously but not necessarily, the closing element is made as a cover **104** placed on top of the chamber **102**.

In the illustrated embodiment, the system **100** comprises a source measure unit or SMU **106** connected to the electrodes **16** and configured for generating a potential difference, which is generally constant, between the electrodes while at the same time measuring the intensity of the electric current flowing across them. In this manner, the SMU **106** allows for indirectly assessing the electric resistance value of the polymeric layer **14**. For example, the SMU **106** may be a Keithley-type unit.

The experimental tests were carried out on the sensor **10** as follows.

For each detection conducted by the sensor, a quantity of approx. 10 ml of analyte was introduced into the chamber **102**. In particular, said quantity of analyte was introduced into the chamber **102,** by means of a syringe, through the cover **104**. The cover **104** was then removed from the rest of the container **100**, and the experimental test continued in ambient conditions.

During these experimental tests, the analyte comprised an aqueous ammonia solution of different molarity within the range of approx. 3.5 mM to approx. 1.5 M. During the measurements, the device **106** was polarized (e.g. polarization voltage set to 5 V) and thus detected a current intensity as a function of time as the analyte was added and removed. All measurements were taken in ambient conditions.

During the experimental tests, the sensing polymeric layer **14**, which preferably consisted of a thin layer or film of PMDA-ODA, was subjected to the vapours developed by an ammonia solution at a concentration of approx. 1.5 M for approx. 300 s, by introducing into the chamber **102** a quantity of approx. 10 ml of analyte. As shown in Figure 2, the introduction of the analyte into the chamber **102** caused a rapid increase in the current intensity flowing across the electrodes **16** connected to the polymeric layer **14**.

This means that, when the sensor **10** remains exposed to the analyte for long periods, a higher current intensity will be detected.

As can be observed in Figure 2, the response of the PMDA-ODA film is not saturated for long periods of exposition to the analyte containing the ammonia solution. In fact, as shown in the graph of Figure 2, the response can be divided into two parts: a first, faster initial response and a second, slower final response. During the faster initial response, it takes approx. 90 s for the current intensity to increase by two orders of magnitude. The increase then becomes much slower during the second, final response.

Subsequently, the sensing polymeric layer **14** was subjected to atmospheric conditions by removing the cover **104** from the rest of the container **100**, thereby opening the chamber **102**.

When the chamber cover is removed, the intensity of the current flowing across the two electrodes **16** in contact with the sensing polymeric layer **14** falls back to a basic value. As in the previous phase, a first, initial recovery phase and a second, final recovery phase can be observed in the current intensity variation. In fact, in the first 40 s following removal of the cover **104**, resulting in the opening of the chamber **102**, during the first, initial recovery phase, the current intensity decreases by approximately two orders of magnitude. Afterwards, during the second, final recovery phase, a very slow and gradual continuous decrease occurs. When 300 s have elapsed since the removal of the cover **104**, the current intensity reaches a value slightly exceeding its "normal" initial value, due to the reaction that has occurred between the ammonia and the polyimide of the polymeric layer **14** with which the electrodes **16** are in contact.

The electric conductivity of the polyimide is of ionic nature, because PMDA-ODA does not have a conjugated double-bond structure. Imides are generally very weak bases; therefore, when they interact with a strong base, such as ammonia, they are very likely to become H+ donors (behaving as a Brønsted-Lowry acid) for the ammonia molecules. Amide deprotonation then follows the resulting formation of NH4+, leading to increased ionic conductivity of the polymer.

Since the analyte used in the experimental tests contained ammonia and water, the response of the PMDA-ODA sensor to water was studied separately in order to understand how that response affects the behaviour in the presence of ammonia. Response to water is significantly lower also when the ammonia-containing solution is very diluted (e.g. down to 0.007 M). Moreover, it has been recently demonstrated that, as ammonia evaporates from the aqueous solution, the temperature of the solution decreases, thus countering the evaporation of water in the time interval during which the experimental tests are carried out. It is therefore believed that water evaporation cannot affect the results and detections obtained from the sensor **10**.

It was also observed that, when the polymeric material is conditioned in an environment with 100% humidity before being used as an ammonia detector, it offers better performance.

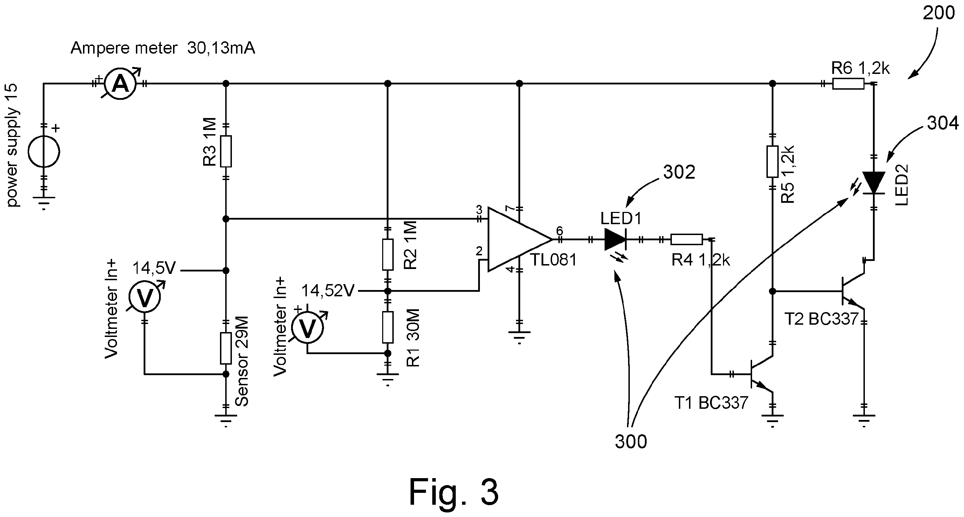

In order to test the feasibility of using a polyimide, such as PMDA-ODA, in the sensor **10**, the system **100** comprises a simple analog circuit **200**, and was designed and manufactured as shown in the non-limiting example of Figure 3.

Said circuit **200** was implemented with the sensor **10** having the polymeric layer **14** deposited on a substrate **12** made of textile material, e.g. cotton (which proves the versatility of the sensor as concerns the material employed in the polymeric region, which can be easily used in combination with many different types of substrates). In particular, the sensor **10** comprises a plurality of polyimide strips deposited on the substrate **12**. For example, the sensor **10** comprises four strips obtained by deposition of 20 µl of PMDA-ODA solution on the substrate **12** - which, as aforementioned, is preferably made of textile, e.g. cotton. Advantageously but not necessarily, the polyimide strips are separated by uncovered cotton bands, thus forming a system of resistors connected in parallel. Polymerization was carried out as previously described in the present description, with no alterations in the cotton textile.

When the ammonia solution is introduced into the chamber **102**, the intensity of the current flowing through the polymeric layer **14**, in particular through said polymeric strips, increases because the electric resistance decreases as previously described.

The output of the circuit **200** is connected to a signalling apparatus **300** outputting a signal perceivable by a user and indicating that the electric resistance of the polymeric layer **14** has crossed (exceeded and/or fallen below) one or more predetermined threshold values.

In particular, the signalling apparatus **300** may include a luminous normal-operation indicator **302**, such as a coloured (e.g. green) LED, configured for signalling that the electric resistance of the polymeric layer **14** exceeds a predetermined normal-operation threshold value indicative of the absence, or substantially negligible concentration, of ammonia.

As an alternative to or in combination with the luminous indicator **302**, the signalling apparatus **300** may also include an additional luminous faulty-operation indicator **304**, such as a LED configured (e.g. red) for signalling that the electric resistance of the polymeric layer **14** is below a predetermined faulty-operation threshold value indicating the presence, or a substantially significant concentration, of ammonia.

Of course, without prejudice to the principle of the invention, the forms of embodiment and the implementation details may be extensively varied from those described and illustrated herein by way of non-limiting example, without however departing from the scope of the invention as set out in the appended claims.

It will, in fact, be apparent to a man skilled in the art that the application range of said sensor is particularly broad.

Ammonia, which is typically produced in large amounts and transported via pipes, railway or trucks, is used in many industrial fields. Some non-limiting examples of industrial fields where ammonia is used are the petrochemical, fertilizer and oil industries. Ammonia sensors are therefore used throughout the ammonia transportation and storage stages in the different industries.

Furthermore, anhydrous ammonia is also used as coolant in large industrial refrigeration plants, especially in the food and drink industry. Many tons of ammonia are used for such purposes. Therefore, reliable systems and sensors are needed for leak detection.

The sensor according to the present invention can thus be used for monitoring and controlling decontamination and possible leaks of ammonia in indoor environments or bounded spaces in technological fields such as:
- food and drink industry,
- poultry, meat or fish processing,
- refrigeration systems,
- storage freezers,
- chemical production,
- aquatic plants, and
- ammonia transportation (gas pipelines, etc.).

## Claims

1. Sensor (**10**) for detecting the presence of gas or vapour;
said sensor (**10**) comprising:
- a substrate (**12**);
- a polymeric layer (**14**) deposited on said substrate (**12**) and including a polyimide having electrically conductive properties, so that the electric resistance of said polyimide is variable according to the quantity of gas or vapour adsorbed by said polyimide; and
- a pair of electrodes (**16**) electrically connected to said polymeric layer (**14**) and configured for allowing the detection of the electric resistance value of said polyimide;
**characterized in that** said electric resistance varies as a function of a concentration of ammonia gas and/or vapour, since said polyimide has imide groups which, when a quantity of ammonia is adsorbed by a region of said polyimide, are capable of reacting with said adsorbed quantity of ammonia to turn into amide groups; and
**in that** said sensor (**10**) further comprises means configured for outputting an indication of the presence of said ammonia gas or vapour based on the detected electric resistance of the polyimide of the polymeric layer (**14**).

2. Sensor according to claim 1, wherein said substrate (**12**) is rigid, and is preferably made of glass or quartz.

3. Sensor according to claim 1, wherein said substrate (**12**) is flexible, and is preferably a textile.

4. Sensor according to any one of the preceding claims, wherein said substrate (**12**) is electrically insulating.

5. Sensor according to any one of the preceding claims, wherein the polyimide of said polymeric layer (**14**) is a condensation polyimide.

6. Sensor according to claim 5, wherein said polyimide comprises PMDA-ODA.

7. Sensor according to claim 6, wherein said polymeric layer (**14**) is made by depositing on said substrate (**12**) a solution including a value in the range of 10% to 20% in weight of pyromellitic dianhydride-co-4,4'-oxydianiline and aminic acid in N-methyl-2-pyrrolidone; said solution being diluted in a mixture of dimethylacetamide and an organic polar solvent, e.g. acetone, at a concentration between 1:0.5 and 1:2; said solution being left to dry on the substrate (**12**) for at least one hour and then polymerized on said substrate (**12**) by oven heating.

8. System comprising a sensor (**10**) according to any one of claims 1 to 7.

9. System according to claim 8, further comprising a source measure unit or SMU (**106**) electrically connected to said electrodes (**16**) and configured for generating a potential difference between said electrodes (**16**) while at the same time measuring the intensity of the electric current flowing across said electrodes (**16**), so as to detect the value of said electric resistance.

10. System according to claim 8 or 9, further comprising a control circuit (**200**) configured for detecting if the electric resistance of the polymeric layer (**14**) has crossed - whether by exceeding or falling below - at least one predetermined threshold value.

11. System according to claim 10, comprising a signalling apparatus (**300**) electrically connected to the output of said control circuit (**200**) and configured for outputting a signal perceivable by a user and indicating that said at least one threshold value has been crossed by said resistance, exceeding and/or falling below said at least one threshold value.

12. Method for manufacturing a sensor (**10**) for detecting the presence of ammonia gas and/or vapour, said method comprising the following operating phases:
- providing a substrate (**12**);
- making a polymeric layer (**14**) deposited on said substrate (**12**) through the following steps:
providing a solution including a value in the range of 10% to 20% in weight of pyromellitic dianhydride-co-4,4'-oxydianiline and aminic acid in N-methyl-2-pyrrolidone,
diluting said solution into a mixture of dimethylacetamide and an organic polar solvent, e.g. acetone, at a concentration between 1:0.5 and 1:2,
depositing said solution on said substrate (**12**),
leaving said solution to dry on said substrate (**12**) for at least one hour, and
polymerizing said solution on said substrate (**12**) by oven heating, thereby obtaining said polymeric layer (**14**) on said substrate (**12**);
- providing a pair of electrodes (**16**) electrically connected to said polymeric layer (**14**) and configured for allowing the detection of the electric resistance value of said polyimide; wherein said electric resistance varies as a function of a concentration of ammonia gas and/or vapour, since said polyimide has imide groups which, when a quantity of ammonia is adsorbed by a region of said polyimide, are capable of reacting with said adsorbed quantity of ammonia to turn into amide groups; and
- providing means configured for outputting an indication of the presence of said ammonia gas or vapour based on the detected electric resistance of the polyimide of the polymeric layer (**14**).

13. Method according to claim 12, wherein polymerization occurs by heating the solution deposited and dried on said substrate (**12**) in an oven in two consecutive heating steps.

14. Method according to claim 13, wherein the first heating step is shorter and occurs at a lower temperature, and the second heating step lasts longer and occurs at a higher temperature.

15. Method according to claim 14, wherein said first heating step lasts for a period of approximately 15-60 minutes and occurs at a temperature between 60°C and 100°C.

16. Method according to claim 15, wherein the first heating step lasts approximately 20 minutes and occurs at a temperature of approximately 80°C.

17. Method according to any one of claims 14 to 16, wherein said second heating step lasts for a period of 120-200 minutes and occurs at a temperature between 170°C and 250°C.

18. Method according to claim 17, wherein the second heating step lasts approximately 150 minutes and occurs at a temperature of approximately 220°C.

19. Use of a sensor for detecting the presence of ammonia gas and/or ammonia vapour; said sensor (**10**) comprising:
- a substrate (**12**);
- a polymeric layer (**14**) deposited on said substrate (**12**) and including a polyimide having electrically conductive properties when such region adsorbs a quantity of ammonia, said polyimide having imide groups capable of reacting with said adsorbed quantity of ammonia to turn into amide groups, so that the electric resistance of said polyimide is variable according to the quantity of ammonia gas or vapour adsorbed by said polyimide; and
- a pair of electrodes (**16**) electrically connected to said polymeric layer (**14**) and configured for allowing the detection of the electric resistance value of said polyimide.

## Patentansprüche

1. Sensor (10) zum Erfassen des Vorhandenseins von Gas oder Dampf; der Sensor (10) umfassend:
- ein Substrat (10)
- eine polymere Schicht (14), die auf dem besagten Substrat (12) aufgebracht ist und ein Polyimid mit elektrisch leitfähigen Eigenschaften enthält, so dass der elektrische Widerstand des besagten Polyimids in Abhängigkeit von der Menge des durch das besagte Polyimid adsorbierten Gases oder Dampfes variabel ist; und
- ein Paar von Elektroden (16), die elektrisch mit der besagten Polymerschicht (14) verbunden und konfiguriert sind, um die Erfassung des elektrischen Widerstandswerts des besagten Polyimids zu ermöglichen;
**dadurch gekennzeichnet, dass** der besagte elektrische Widerstand als eine Funktion der Konzentration von Ammoniakgas und/oder -dampf variiert, da das besagte Polyimid Imidgruppen aufweist, die, wenn eine Menge Ammoniak durch einen Bereich des besagten Polyimids adsorbiert wird, in der Lage sind, mit der besagten adsorbierten Menge Ammoniak zu reagieren, um sich in Amidgruppen umzuwandeln; und
dass der besagte Sensor (10) ferner Mittel umfasst, die konfiguriert sind für Ausgabe einer Anzeige des Vorhandenseins des besagten Ammoniakgases oder -dampfes basierend auf dem festgestellten elektrischen Widerstand des Polyimids der Polymerschicht (14).

2. Sensor nach Anspruch 1, wobei das besagte Substrat (12) starr ist, und vorzugsweise aus Glas oder Quarz hergestellt ist.

3. Sensor nach Anspruch 1, wobei das besagte Substrat (12) flexibel ist, und vorzugsweise ein Textil ist.

4. Sensor nach einem der vorhergehenden Ansprüche, wobei das besagte Substrat (12) elektrisch isolierend ist.

5. Sensor nach einem der vorhergehenden Ansprüche, wobei das Polyimid der besagten Polymerschicht (14) ein Kondensationspolyimid ist.

6. Sensor nach Anspruch 5, wobei das besagte Polyimid PMDA-ODA umfasst.

7. Sensor nach Anspruch 6, wobei die besagte Polymerschicht (14) durch Aufbringen einer Lösung auf das besagte Substrat (12) hergestellt wird, die einen Wert im Bereich von 10 bis 20 Gew.-% Pyromellitsäuredianhydrid-co-4, 4'-Oxydianilin und Aminosäure in N-Methyl-2-pyrrolidon enthält; wobei die besagte Lösung in einer Mischung aus Dimethylacetamid und einem organischen polaren Lösungsmittel verdünnt wird, z.B. Aceton, in einer Konzentration zwischen 1:0,5 und 1:2 verdünnt wird; wobei die besagte Lösung mindestens eine Stunde lang auf dem Substrat (12) trocknen gelassen und dann durch Ofenerhitzung auf dem Substrat (12) polymerisiert wird.

8. System umfassend einen Sensor (10) nach einem der Ansprüche 1 bis 7.

9. System nach Anspruch 8, weiter umfassend eine Quellenmesseinheit oder SMU (106), die elektrisch mit den besagten Elektroden (16) verbunden ist und konfiguriert ist, um eine Potentialdifferenz zwischen den besagten Elektroden (16) zu erzeugen, während sie gleichzeitig die Intensität des elektrischen Stroms misst, der über die besagten Elektroden (16) fließt, um so den Wert des besagten elektrischen Widerstands zu erfassen.

10. System nach Anspruch 8 oder 9, weiter umfassend eine Steuerschaltung (200) konfiguriert zum Erfassen, ob der elektrische Widerstand der Polymerschicht (14) - sei es durch Über- oder Unterschreiten - mindestens einen vorbestimmten Schwellenwert durchschritten hat.

11. System nach Anspruch 10, umfassend eine Signalisierungsvorrichtung (300), die elektrisch mit dem Ausgang der besagten Steuerschaltung (200) verbunden ist und konfiguriert ist, um ein Signal auszugeben, das von einem Benutzer wahrnehmbar ist und anzeigt, dass der besagte mindestens eine Schwellenwert von dem besagten Widerstand durchschritten wurde, wobei der besagte mindestens eine Schwellenwert über- und/oder unterschritten wurde.

12. Verfahren zur Herstellung eines Sensors (10) zum Erfassen des Vorhandenseins von Ammoniakgas und/oder -dampf, besagtes Verfahren umfassend die folgenden Betriebsphasen:
- Bereitstellen eines Substrats (12);
- Herstellen einer auf dem besagten Substrat (12) aufgebrachten Polymerschicht (14) durch die folgenden Schritte:
Bereitstellen einer Lösung, die einen Wert im Bereich von 10 bis 20 Gew.-% Pyromellitsäuredianhydrid-co-4, 4'-Oxydianilin und Aminsäure in N-Methyl-2-pyrrolidon umfasst,
Verdünnen der Lösung in ein Gemisch aus Dimethylacetamid und einem organischen polaren Lösungsmittel, z.B. Aceton, in einer Konzentration zwischen 1:0,5 und 1:2, Aufbringen der besagten Lösung auf das besagte Substrat (12), Trocknenlassen der besagten Lösung auf dem besagten Substrat (12) für mindestens eine Stunde, und Polymerisieren der besagten Lösung auf dem besagten Substrat (12) durch Erhitzen im Ofen, wodurch die besagte Polymerschicht (14) auf dem besagten Substrat (12) erhalten wird;
- Bereitstellen eines Paars von Elektroden (16), die elektrisch mit der besagten Polymerschicht (14) verbunden und so konfiguriert sind, dass sie die Erfassung des elektrischen Widerstandswerts des besagten Polyimids ermöglichen; wobei der besagte elektrische Widerstand als Funktion einer Konzentration von Ammoniakgas und/oder -dampf variiert, da das besagte Polyimid Imidgruppen aufweist, die, wenn eine Menge Ammoniak durch einen Bereich des besagten Polyimids adsorbiert wird, in der Lage sind, mit der besagten adsorbierten Menge Ammoniak zu reagieren, um sich in Amidgruppen umzuwandeln; und
- Bereitstellen von Mittels, die zur Ausgabe einer Anzeige des Vorhandenseins des besagten Ammoniakgases oder-dampfes auf der Grundlage des erfassten elektrischen Widerstands des Polyimids der besagten Polymerschicht (14) konfiguriert sind.

13. Verfahren nach Anspruch 12, wobei die Polymerisation durch Erhitzen der auf dem Substrat (12) aufgetragenen und getrockneten Lösung in einem Ofen in zwei aufeinanderfolgenden Erhitzungsschritten erfolgt.

14. Verfahren nach Anspruch 13, wobei der erste Erwärmungsschritt kürzer ist und bei einer niedrigeren Temperatur erfolgt, und der zweite Erwärmungsschritt länger dauert und bei einer höheren Temperatur erfolgt.

15. Verfahren nach Anspruch 14, wobei der erste Erhitzungsschritt für einen Zeitraum von etwa 15-60 Minuten dauert und bei einer Temperatur zwischen 60°C und 100°C erfolgt.

16. Verfahren nach Anspruch 15, wobei der erste Erwärmungsschritt etwa 20 Minuten dauert und bei einer Temperatur von etwa 80°C erfolgt.

17. Verfahren nach einem der Ansprüche 14 bis 16, wobei der besagte zweite Erhitzungsschritt über einen Zeitraum von 120-200 Minuten dauert und bei einer Temperatur zwischen 170°C und 250°C erfolgt.

18. Verfahren nach Anspruch 17, wobei der zweite Erhitzungsschritt etwa 150 Minuten dauert und bei einer Temperatur von etwa 220°C erfolgt.

19. Verwendung eines Sensors zum Erfassen des Vorhandenseins von Ammoniakgas und/oder Ammoniakdampf; besagter Sensor (10) umfassend:
- ein Substrat (12);
- eine besagte Polymerschicht (14), die auf dem besagten Substrat (12) aufgebracht ist und ein Polyimid umfasst, das elektrisch leitende Eigenschaften aufweist, wenn ein solcher Bereich eine Menge Ammoniak adsorbiert, wobei das besagte Polyimid Imidgruppen aufweist, die in der Lage sind, mit der besagten adsorbierten Menge Ammoniak zu reagieren, um sich in Amidgruppen umzuwandeln, so dass der elektrische Widerstand des besagten Polyimids entsprechend der Menge Ammoniakgas oder-dampf, die von dem besagten Polyimid adsorbiert wird, variabel ist; und
- ein Paar von Elektroden (16), die elektrisch mit der besagten Polymerschicht (14) verbunden und so konfiguriert sind, dass sie die Erfassung des elektrischen Widerstandswertes des besagten Polyimids ermöglichen.

## Revendications

1. Capteur (10) pour détecter la présence de gaz ou de vapeur ;
ledit capteur (10) comprenant :
- un substrat (12) ;
- une couche polymère (14) déposée sur ledit substrat (12) et comportant un polyimide ayant des propriétés électroconductrices, de telle sorte que la résistance électrique dudit polyimide est variable en fonction de la quantité de gaz ou de vapeur adsorbée par ledit polyimide ; et
- une paire d'électrodes (16) électriquement raccordées à ladite couche polymère (14) et configurée pour permettre la détection de la valeur de résistance électrique dudit polyimide ;
**caractérisé en ce que** ladite résistance électrique varie en fonction d'une concentration de gaz et/ou de vapeur d'ammoniac, étant donné que ledit polyimide a des groupes imide qui, lorsqu'une quantité d'ammoniac est adsorbée par une région dudit polyimide, sont capables de réagir avec ladite quantité adsorbée d'ammoniac pour se transformer en groupes amide ; et
**en ce que** ledit capteur (10) comprend en outre un moyen configuré pour émettre une indication de la présence desdits gaz ou vapeur d'ammoniac sur la base de la résistance électrique détectée du polyimide de la couche polymère (14).

2. Capteur selon la revendication 1, dans lequel ledit substrat (12) est rigide, et est de préférence en verre ou en quartz.

3. Capteur selon la revendication 1, dans lequel ledit substrat (12) est souple, et est de préférence un textile.

4. Capteur selon l'une quelconque des revendications précédentes, dans lequel ledit substrat (12) est électriquement isolant.

5. Capteur selon l'une quelconque des revendications précédentes, dans lequel le polyimide de ladite couche polymère (14) est un polyimide de condensation.

6. Capteur selon la revendication 5, dans lequel ledit polyimide comprend du PMDA-ODA.

7. Capteur selon la revendication 6, dans lequel ladite couche polymère (14) est faite par dépôt sur ledit substrat (12) d'une solution comportant une valeur dans la plage de 10 % à 20 % en poids de dianhydride pyromellitique-co-4,4'-oxydianiline et d'acide aminique dans de la N-méthyl-2-pyrrolidone ; ladite solution étant diluée dans un mélange de diméthylacétamide et d'un solvant polaire organique, par exemple l'acétone, en une concentration entre 1/0,5 et 1/2 ; ladite solution étant laissée à sécher sur le substrat (12) pendant au moins une heure puis polymérisée sur ledit substrat (12) par chauffage au four.

8. Système comprenant un capteur (10) selon l'une quelconque des revendications 1 à 7.

9. Système selon la revendication 8, comprenant en outre une unité de source et de mesure ou SMU (106) électriquement raccordée auxdites électrodes (16) et configurée pour générer une différence de potentiel entre lesdites électrodes (16) tout en mesurant en même temps l'intensité du courant électrique s'écoulant à travers lesdites électrodes (16), de manière à détecter la valeur de ladite résistance électrique.

10. Système selon la revendication 8 ou 9, comprenant en outre un circuit de commande (200) configuré pour détecter si la résistance électrique de la couche polymère (14) a franchi - soit en dépassant soit en tombant au-dessous - au moins une valeur seuil prédéterminée.

11. Système selon la revendication 10, comprenant un appareil de signalisation (300) électriquement raccordé à la sortie dudit circuit de commande (200) et configuré pour émettre un signal perceptible par un utilisateur et indiquer que ladite au moins une valeur seuil a été franchie par ladite résistance, en dépassant et/ou en tombant au-dessous de ladite au moins une valeur seuil.

12. Procédé de fabrication d'un capteur (10) pour détecter la présence de gaz et/ou vapeur d'ammoniac, ledit procédé comprenant les phases de fonctionnement suivantes :
- fournir un substrat (12) ;
- fabriquer une couche polymère (14) déposée sur ledit substrat (12) par l'intermédiaire des étapes suivantes :
fournir une solution comportant une valeur dans la plage de 10 % à 20 % en poids de dianhydride pyromellitique-co-4,4'-oxydianiline et d'acide aminique dans de la N-méthyl-2-pyrrolidone,
diluer ladite solution étant dans un mélange de diméthylacétamide et d'un solvant polaire organique, par exemple l'acétone, en une concentration entre 1/0,5 et 1/2, déposer ladite solution sur ledit substrat (12),
laisser ladite solution sécher sur ledit substrat (12) pendant au moins une heure, et
polymériser ladite solution sur ledit substrat (12) par chauffage au four, permettant ainsi d'obtenir ladite couche polymère (14) sur ledit substrat (12) ;
- fournir une paire d'électrodes (16) électriquement reliées à ladite couche polymère (14) et configurées pour permettre la détection de la valeur de résistance électrique dudit polyimide ; dans lequel ladite résistance électrique varie en fonction d'une concentration de gaz et/ou de vapeur d'ammoniac, étant donné que ledit polyimide a des groupes imides qui, lorsqu'une quantité d'ammoniac est adsorbée par une région dudit polyimide, sont capables de réagir avec ladite quantité adsorbée d'ammoniac pour se transformer en groupes amide ; et
- fournir un moyen configuré pour émettre une indication de la présence desdits gaz ou vapeur d'ammoniac sur la base de la résistance électrique détectée du polyimide de la couche polymère (14).

13. Procédé selon la revendication 12, dans lequel la polymérisation se produit par chauffage de la solution déposée et séchée sur ledit substrat (12) dans un four en deux étapes de chauffage consécutives.

14. Procédé selon la revendication 13, dans lequel la première étape de chauffage est plus courte et se produit à une température inférieure, et la seconde étape de chauffage dure plus longtemps et se produit à une température supérieure.

15. Procédé selon la revendication 14, dans lequel ladite première étape de chauffage dure pendant une période d'approximativement 15 à 60 minutes et se produit à une température entre 60 °C et 100 °C.

16. Procédé selon la revendication 15, dans lequel la première étape de chauffage dure approximativement 20 minutes et se produit à une température d'approximativement 80 °C.

17. Procédé selon l'une quelconque des revendications 14 à 16, dans lequel ladite seconde étape de chauffage dure au moins pendant une période de 120 à 200 minutes et se produit à une température comprise entre 170 °C et 250 °C.

18. Procédé selon la revendication 17, dans lequel la seconde étape de chauffage dure approximativement 150 minutes et se produit à une température d'approximativement 220 °C.

19. Utilisation d'un capteur pour détecter la présence de gaz d'ammoniac et/ou de vapeur d'ammoniac ; ledit capteur (10) comprenant :
- un substrat (12) ;
- une couche polymère (14) déposée sur ledit substrat (12) et comportant un polyimide ayant des propriétés électroconductrices lorsqu'une telle région adsorbe une quantité d'ammoniac, ledit polyimide ayant des groupes imide capables de réagir avec ladite quantité adsorbée d'ammoniac pour se transformer en groupes amide, de manière à ce que la résistance électrique dudit polyimide soit variable en fonction de la quantité de gaz ou vapeur d'ammoniac adsorbée par ledit polyimide ; et
- une paire d'électrodes (16) électriquement raccordées à ladite couche polymère (14) et configurées pour permettre la détection de la valeur de résistance électrique dudit polyimide.
